# EUROPEAN PATENT SPECIFICATION

(11) **EP 3 824 882 B1**
(45) Date of publication and mention of the grant of the patent: **01.01.2025**
(21) Application number: 19838422.4
(22) Date of filing: 17.05.2019
(51) Int. Cl.: A61K 9/70, A61K 47/34, C09J 7/38, C09J 11/02, C09J 183/05, C09J 183/06, C09J 183/07

(54) **ADDITION REACTION-CURABLE SILICONE ADHESIVE COMPOSITION FOR SKIN PATCH AND ADHESIVE TAPE FOR SKIN PATCH**
DURCH ADDITIONSREAKTION HÄRTBARE SILIKONKLEBSTOFFZUSAMMENSETZUNG FÜR HAUTPFLASTER UND KLEBEBAND FÜR HAUTPFLASTER
COMPOSITION D'ADHÉSIF DE SILICONE DURCISSABLE PAR RÉACTION D'ADDITION POUR TIMBRE CUTANÉ ET BANDE ADHÉSIVE POUR TIMBRE CUTANÉ

(30) Priority: 17.07.2018 JP 2018134505
(43) Date of publication of application: 26.05.2021
(73) Proprietor: Shin-Etsu Chemical Co., Ltd., Tokyo 100-0005 (JP)
(72) Inventor: KOBAYASHI Akihiro, Annaka-shi, Gunma 379-0224 (JP); KURODA Yasuyoshi, Annaka-shi, Gunma 379-0224 (JP); AOKI Shunji, Annaka-shi, Gunma 379-0224 (JP)
(74) Representative: Wibbelmann, Jobst
(86) International application number: PCT/JP2019/019701
(87) International publication number: WO 2020/017142

(56) References cited:
- EP-A1- 2 524 954
- WO-A1-2017/129429
- WO-A1-2018/190047
- WO-A1-2019/087759
- JP-A- 2004 067 720
- JP-A- 2008 274 251
- JP-A- 2009 274 959
- JP-A- 2011 001 448
- JP-A- 2016 194 028
- JP-A- 2017 222 819

## Description

### TECHNICAL FIELD

The present invention relates to an addition reaction-curable silicone adhesive composition for skin application, and an adhesive tape for skin application that uses a cured product of this composition.

### BACKGROUND ART

The main chain of polysiloxane for constituting a silicone adhesive is composed of Si-O bonds which have high bonding energy. This makes polysiloxane excellent in heat resistance, cold resistance, weather resistance, electric insulation property, and chemical resistance. Hence, such polysiloxanes are used under severe environments in the form of heat resistant tape, electric insulating tape, masking tape for various processes, flame retardant mica tape, and so forth. Because of air permeability and low irritation, silicone adhesives are attached to skin for use, too.

For skin attachment usage, many acrylic or rubber-based adhesives have been used. However, these adhesives have such high adhesive strength to skin or other adherends that when the adhesives are peeled off, skin keratin is excessively removed or body hair is pulled, consequently causing pain or discomfort. Further, the excessive irritation to skin may cause secondary inflammation.

To solve such problems, studies have been conducted using silicone adhesives for skin attachment application. In consideration of the adhesion properties upon attachment, these silicone adhesives may be used with a peroxide and a platinum catalyst for crosslinking, or the silicone adhesives may be used without crosslinking (Patent Document 1).

However, crosslinking by the method as described above results in sufficient cohesion, but lowers the inherent adhesion properties (tackiness, adhesive strength) of the silicone adhesives, and also lowers the suitability as the adhesive for skin application. On the other hand, not performing crosslinking results in insufficient cohesion, so that some adhesive remains on the skin surface even after the peeling.

Meanwhile, in comparison with acrylic or rubber-based adhesives, silicone adhesives are characterized by less removal of body hair and the stratum corneum of skin when peeled. Nevertheless, there is a problem that the adhesive strength is decreased when the peeled silicone adhesives are attached again.

Silicone adhesives are used in medical tapes such as surgical tape and dressing material, for example. In these usages, silicone adhesives are temporarily peeled for re-attachment or observation. The decrease in adhesive strength due to these re-attachments is not desirable. Patent Document 2 discloses adhesives for skin application which comprise a divinyl-terminated polysiloxane and a MMᵥᵢQ resin having OH groups.

As noted above, there have been demands for the development of a silicone adhesive for skin application that prevents a decrease in adhesive strength over time, also suppresses a decrease in the adhesive strength due to re-attachment, and has excellent reworkability.

### CITATION LIST

### PATENT LITERATURE

Patent Document 1: JP H03-200885 A
Patent Document 2: WO 2017/129429 A1

### SUMMARY OF INVENTION

### TECHNICAL PROBLEM

The present invention has been made in view of the above-described circumstances. An object of the present invention is to provide: an addition reaction-curable silicone adhesive composition for skin application that forms an adhesive layer which has suitable adhesive strength to human skin, suppresses a decrease in the adhesive strength when re-attached, and exhibits excellent reworkability and durability; and an adhesive tape for skin application that uses a cured product of the composition.

### SOLUTION TO PROBLEM

To achieve the object, the present invention provides an addition-curable silicone adhesive composition for skin application, comprising the following components (X), (C), and (D):
(X) 100 parts by mass of a mixture of the following component (A) with the following component (B), or a condensation reaction product between the following component (A) and the following component (B),
(A) 55 to 90 parts by mass of a diorganopolysiloxane consisting of the following components (A1) and (A2), wherein a mass ratio of (A1)/(A2) is within a range from 90/10 to 15/85,
   (A1) a diorganopolysiloxane having two or more alkenyl groups per molecule and an alkenyl group-content of at least 0.0005 mol/100 g and less than 0.15 mol/100 g, wherein the molecular chain terminals are not completely blocked in that some SiOH groups are left remaining, and
   (A2) a diorganopolysiloxane having a SiOH group at a terminal and no alkenyl group, and
(B) 10 to 45 parts by mass of an organopolysiloxane, provided that a total of the components (A) and (B) is 100 parts by mass, wherein the organopolysiloxane contains an R¹₃SiO_{0.5} unit, a SiO₂ unit, and a siloxane unit having a silicon atom-bonded hydroxyl group and/or a siloxane unit having a silicon atom-bonded alkoxy group with 1 to 6 carbon atoms, each R¹ independently represents a monovalent hydrocarbon group having 1 to 10 carbon atoms, and a molar ratio of the R¹₃SiO_{0.5} unit/the SiO₂ unit is 0.5 to 1.5;
(C) an organohydrogenpolysiloxane containing three or more SiH groups per molecule, wherein the organohydrogenpolysiloxane is in such an amount that a molar ratio of the SiH groups in the component (C) to the alkenyl groups in the component (A) is 0.2 to 15; and
(D) a platinum group metal-based catalyst in an amount of 1 to 5,000 ppm in terms of platinum group metal relative to the total amount of the components (A) and (B) based on mass.

The inventive addition-curable silicone adhesive composition for skin application is capable of forming an adhesive layer which has suitable adhesive strength to human skin, suppresses a decrease in the adhesive strength when re-attached, and exhibits excellent reworkability and durability.

More preferably, the present invention further comprises the following component (E) in an amount of 25 to 900 parts by mass relative to 100 parts by mass of the total of the components (A) and (B),
(E) at least one aliphatic hydrocarbon solvent selected from the group consisting of hexane, heptane, isooctane, octane, decane, cyclohexane, methylcyclohexane, and isoparaffin with a boiling point ranging from 115°C to 138°C.

This enables the inventive addition-curable silicone adhesive composition for skin application to have preferable concentration and viscosity.

Moreover, in the present invention, the component (C) is preferably an organohydrogenpolysiloxane shown by the following formula (1), 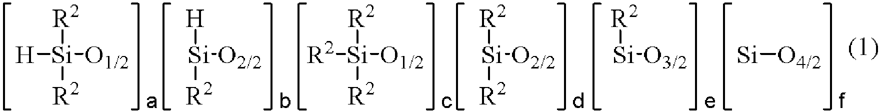 wherein each R² is identical to or different from one another and represents a monovalent hydrocarbon group having 1 to 10 carbon atoms and no aliphatic unsaturated bond, "a" represents an integer of 0 or more, "b" represents an integer of 1 or more, "c" represents an integer of 0 or more, "d" represents an integer of 0 or more, "e" represents an integer of 0 or more, and "f" represents an integer of 0 or more, with a+b≥3 and 3≤a+b+c+d+e+f≤1,000.

This makes it possible to form an adhesive layer which has more suitable adhesive strength to human skin, more suppresses a decrease in the adhesive strength when re-attached, and exhibits more excellent reworkability and durability.

Further preferably, in the present invention, the component (A) is in such an amount that the mass ratio of (A1)/(A2) is within a range from 75/25 to 25/75.

This makes it possible to form an adhesive layer which has further suitable adhesive strength to human skin, further suppresses a decrease in the adhesive strength when re-attached, and exhibits further excellent reworkability and durability.

Further preferably, in the present invention, the component (X) is a condensation reaction product among the components (A1), (A2), and (B), or a mixture of the component (A1) with a condensation reaction product between the components (A2) and (B).

This makes it possible to form an adhesive layer which has still further suitable adhesive strength to human skin, still further suppresses a decrease in the adhesive strength when re-attached, and exhibits still further excellent reworkability and durability.

The present invention preferably further comprises an addition reaction inhibitor as a component (F) in an amount of 0.05 to 8 parts by mass relative to 100 parts by mass of the total of the components (A) and (B).

This can prevent a treatment solution containing the silicone adhesive composition from thickening or gelling before heat curing, for example, in preparing the silicone adhesive composition or applying the silicone adhesive composition to a substrate.

In addition, the present invention provides an adhesive tape for skin application, comprising:
a substrate; and
a cured product layer laminated on at least one side of the substrate, wherein
the cured product layer is formed of a cured product of the above-described silicone adhesive composition.

In the inventive adhesive tape for skin application, since the cured product layer is made of a cured product of the silicone adhesive composition, the inventive adhesive tape has suitable adhesive strength to human skin, can fix the substrate to skin, also suppresses a decrease in the adhesive strength when re-attached, and is excellent in reworkability and durability.

Additionally, the present invention provides a use of the adhesive tape for skin application described above, the use comprising attaching the adhesive tape for skin application to skin.

According to the inventive use of the adhesive tape for skin application, the adhesive tape for skin application is gently usable with respect to human skin.

### ADVANTAGEOUS EFFECTS OF INVENTION

As described above, the inventive addition reaction-curable silicone adhesive composition for skin application has suitable adhesive strength to human skin and consequently can fix a substrate to skin. Further, it is possible to provide an adhesive tape for skin application that suppresses a decrease in the adhesive strength upon re-attachment and is excellent in reworkability and durability.

### DESCRIPTION OF EMBODIMENTS

As described above, there have been demands for the development of a silicone adhesive for skin application that prevents a decrease in adhesive strength over time, also suppresses a decrease in the adhesive strength due to re-attachment, and has excellent reworkability.

The present inventors has earnestly studied the above object and consequently found that when an addition-curable silicone adhesive composition for skin application is prepared to contain a diorganopolysiloxane component (A) consisting of specific components (A1) and (A2) with a mass ratio of (A1)/(A2) in a specific range, a specific organopolysiloxane component (B), an organohydrogenpolysiloxane component, and a platinum group metal-based catalyst component by controlling the ratio between the components (A) and (B) as well as the ratio between the components (A1) and (A2), a silicone adhesive soft but having cohesion and reworkability can be obtained. This finding has led to the completion of the present invention.

Specifically, the present invention is an addition-curable silicone adhesive composition for skin application, comprising the following components (X), (C), and (D):
(X) 100 parts by mass of a mixture of the following component (A) with the following component (B), or a condensation reaction product between the following component (A) and the following component (B),
   (A) 55 to 90 parts by mass of a diorganopolysiloxane consisting of the following components (A1) and (A2), wherein a mass ratio of (A1)/(A2) is within a range from 90/10 to 15/85,
      (A1) a diorganopolysiloxane having two or more alkenyl groups per molecule and an alkenyl group-content of at least 0.0005 mol/100 g and less than 0.15 mol/100 g, wherein the molecular chain terminals are not completely blocked in that some SiOH groups are left remaining, and
      (A2) a diorganopolysiloxane having a SiOH group at a terminal and no alkenyl group, and
   (B) 10 to 45 parts by mass of an organopolysiloxane, provided that a total of the components (A) and (B) is 100 parts by mass, wherein the organopolysiloxane contains an R¹₃SiO_{0.5} unit, a SiO₂ unit, and a siloxane unit having a silicon atom-bonded hydroxyl group and/or a siloxane unit having a silicon atom-bonded alkoxy group with 1 to 6 carbon atoms, each R¹ independently represents a monovalent hydrocarbon group having 1 to 10 carbon atoms, and a molar ratio of the R¹₃SiO_{0.5} unit/the SiO₂ unit is 0.5 to 1.5;
   (C) an organohydrogenpolysiloxane containing three or more SiH groups per molecule, wherein the organohydrogenpolysiloxane is in such an amount that a molar ratio of the SiH groups in the component (C) to the alkenyl groups in the component (A) is 0.2 to 15; and
   (D) a platinum group metal-based catalyst in an amount of 1 to 5,000 ppm in terms of platinum group metal relative to the total amount of the components (A) and (B) based on mass.

Hereinbelow, the present invention will be described in detail, but the present invention is not limited thereto. Note that, hereinafter, the term "addition reaction-curable silicone adhesive composition for skin application" may be simply referred to as "silicone adhesive composition".

### <Silicone Adhesive Composition>

A silicone adhesive composition of the present invention is an addition-curable silicone adhesive composition for skin application containing the following components (X), (C), and (D) as essential component.
(X) a mixture of the following component (A) with the following component (B), or a condensation reaction product between the following component (A) and the following component (B): 100 parts by mass.
(A) a diorganopolysiloxane consisting of the following components (A1) and (A2) with a mass ratio of (A1)/(A2) ranging from 90/10 to 15/85: 55 to 90 parts by mass.
   (A1) a diorganopolysiloxane having two or more alkenyl groups per molecule with the alkenyl group-content of at least 0.0005 mol/100 g and less than 0.15 mol/100 g, wherein the molecular chain terminals are not completely blocked in that some SiOH groups are left remaining.
   (A2) a diorganopolysiloxane having a SiOH group at a terminal and having no alkenyl group.
(B) an organopolysiloxane containing an R¹₃SiO_{0.5} unit (each R¹ independently represents a monovalent hydrocarbon group having 1 to 10 carbon atoms), a SiO₂ unit, and a siloxane unit having a silicon atom bonded to a hydroxyl group and/or a siloxane unit having a silicon atom bonded to an alkoxy group with 1 to 6 carbon atoms, with a molar ratio of the R¹₃SiO_{0.5} unit/the SiO₂ unit being 0.5 to 1.5: 10 to 45 parts by mass (provided that the total of the components (A) and (B) is 100 parts by mass).
(C) an organohydrogenpolysiloxane containing three or more SiH groups per molecule: the amount is such that the molar ratio of the SiH groups in the component (C) to the alkenyl groups in the component (A) is 0.2 to 15.
(D) a platinum group metal-based catalyst: 1 to 5,000 ppm in terms of mass of platinum group metal relative to the total amount of the components (A) and (B) .

In addition to the above components, the inventive silicone adhesive composition may contain an aliphatic hydrocarbon solvent as a component (E), an addition reaction inhibitor as a component (F), and another optional component(s), as necessary. Hereinbelow, each component will be described in detail.

### <Component (X)>

The inventive addition-curable silicone adhesive composition for skin application contains the component (X) as an essential component which is a mixture of the components (A) and (B), or a condensation reaction product of the components (A) and (B). Herein, the condensation reaction product may contain a partially condensed reaction product of the components (A) and (B), and may contain an excessive component of either the component (A) or (B). Moreover, the condensation reaction product may contain unreacted materials of the components (A) and (B). Further, the component (A) consists of the components (A1) and (A2). Herein, the mixture of the components (A) and (B) is equivalent to a mixture of the components (A1), (A2), and (B), and the condensation reaction product between the components (A) and (B) is a product obtained by condensation reaction between the components (A) and (B). In this case also, the condensation reaction product may contain a partially condensed reaction product of the component (B) and the component (A1) and/or the component (A2), and may contain unreacted materials of the components (A1), (A2), and (B). Hereinafter, the component (A) (the components (A1), (A2)), the component (B), and mixtures and condensation reaction products thereof will be described.

### <Component (A)>

The component (A) refers to diorganopolysiloxanes including: a diorganopolysiloxane (A1) which has two or more alkenyl groups per molecule, and in which the alkenyl group-content is not less than 0.0005 mol/100 g but less than 0.15 mol/100 g; and a diorganopolysiloxane (A2) which has a SiOH group at a terminal but has no alkenyl group. The mass ratio of (A1)/(A2) is within a range from 90/10 to 15/85. The mass ratio (A1)/(A2) is preferably 80/20 to 20/80, further preferably 75/25 to 25/75. If the mass ratio (A1)/(A2) is less than 15/85, the crosslinking density in a cured product of the resulting silicone adhesive composition is decreased, and the cohesion becomes insufficient, so that the adhesive will remain on the skin surface after peeling. Meanwhile, if the mass ratio exceeds 90/10, the crosslinking density in a cured product of the resulting silicone adhesive composition is increased, and the adhesion properties (tackiness, adhesive strength) are decreased, and the suitability of the adhesive for skin application is decreased.

The component (A1) is a diorganopolysiloxane having two or more alkenyl groups per molecule, and the alkenyl group-content is at least 0.0005 mol/100 g and less than 0.15 mol/100 g. The component (A1) is a diorganopolysiloxane in which the molecular chain terminals are not completely blocked (i.e., some SiOH groups are left remaining). One kind of the component (A1) may be used alone, or two or more kinds thereof may be used in combination. Examples of the component (A1) include ones shown by the following formula (2). 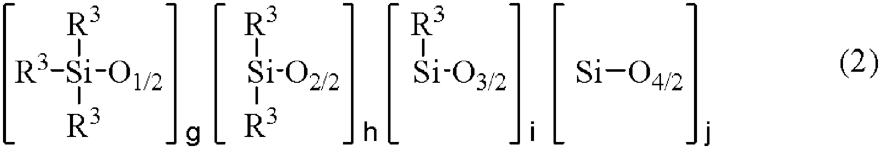 In the formula, each R³ is identical to or different from one another and represents a monovalent hydrocarbon group having 1 to 10 carbon atoms. At least two R³'s are alkenyl group-containing organic groups with 2 to 10 carbon atoms. "g" represents an integer of 2 or more, "h" represents an integer of 1 or more, "i" represents an integer of 0 or more, and "j" represents an integer of 0 or more, with 100≤g+h+i+j≤20,000.

In this formula, R³'s are identical or different monovalent hydrocarbon groups each having 1 to 10 carbon atoms. Among R³'s, at least two are each an alkenyl group-containing organic group having 2 to 10 carbon atoms. Examples of the monovalent hydrocarbon group include alkyl groups such as a methyl group, an ethyl group, a propyl group, and a butyl group; cycloalkyl groups such as a cyclohexyl group; aryl groups such as a phenyl group; etc. Further, some or all of hydrogen atoms bonded to carbon atoms of these groups may be substituted with a halogen atom or another group. Examples of such a substituent include a trifluoromethyl group, a 3,3,3-trifluoropropyl group, etc. Particularly, the substituent is preferably a saturated aliphatic group or an aromatic group, and a methyl group and a phenyl group are preferable. When a phenyl group is incorporated as R³, the phenyl group content is preferably 0.1 to 30 mol% relative to all the organic groups bonded to the silicon atoms in the diorganopolysiloxane of the formula (2). When the content is within this range, the resulting silicone adhesive layer has preferable adhesive strength with respect to human skin.

The alkenyl group-containing organic group preferably has 2 to 10 carbon atoms, and examples thereof include alkenyl groups such as a vinyl group, an allyl group, a hexenyl group, and an octenyl group; acryloylalkyl groups such as an acryloylpropyl group, an acryloylmethyl group, and a methacryloylpropyl group, and a methacryloylalkyl group; cycloalkenylalkyl groups such as a cyclohexenylethyl group; alkenyloxyalkyl groups such as a vinyloxypropyl group; etc. Above all, a vinyl group is preferable from industrial viewpoint.

The amount of the alkenyl groups contained in the component (A1) is 0.0005 moles or more and less than 0.15 moles, more preferably 0.0007 to 0.13 moles, further preferably 0.001 to 0.10 moles, in 100 g of the organopolysiloxane. If the amount is smaller than 0.0005 moles, the curability or the holding power may be lowered. Meanwhile, if the amount is 0.15 moles or more, the resulting adhesive layer is hard and does not have appropriate adhesive strength or tackiness. Normally, this alkenyl group amount can be measured by Hanus method using iodine bromide (the same applies hereinafter) .

When the component (A1) is a diorganopolysiloxane shown by the formula (2), the formula (2) is such that "g" is an integer of 2 or more, "h" is an integer of 1 or more, "i" is an integer of 0 or more, and "j" is an integer of 0 or more, with 100≤g+h+i+j≤20,000, preferably 150≤g+h+i+j≤15,000. When g+h+i+j is 100 or more, the crosslinking points are not so many, and the reactivity is not lowered. When g+h+i+j is 20,000 or less, the viscosity of the composition is not too high and the composition is easily stirred and mixed, so that the handleability is favorable.

The component (A1) can be prepared by subjecting cyclic low-molecular-weight siloxane such as octamethylcyclotetrasiloxane to ring-opening polymerization using a catalyst. In this case, the reaction product after the polymerization contains the raw-material cyclic low-molecular-weight siloxane. For this reason, it is preferable to use the reaction product from which the raw material has been distilled off by heating under reduced pressure while an inert gas is being passed through the reaction product.

Moreover, the amount of a hydroxyl group bonded to a silicon atom (SiOH group) contained in the component (A1) is preferably 0 to 0.45 mass%, particularly preferably 0 to 0.40 mass%, in the component (A). This SiOH group amount can be normally measured by known methods such as Grignard method or NMR method (the same applies hereinafter).

The diorganopolysiloxane component (A1) may be in a form of oil or raw rubber. When the component (A1) is an oil at 25°C, the viscosity is preferably 1,000 to 1,000,000 mPa·s, particularly preferably 5,000 to 800,000 mPa·s. In the case of raw rubber, the viscosity of a solution obtained by dissolving the raw rubber in toluene to make the concentration at 30 mass% is preferably 1,000 to 100,000 mPa·s, particularly preferably 3,000 to 80,000 mPa·s. When the viscosity is at the lower limit value or more, the curability of the adhesive composition and the holding power are not lowered. The viscosity at the upper limit value or less is preferable because the adhesive composition does not have too high viscosity, and the composition is easily stirred when prepared. Note that, in this description, the viscosity refers to a value measured using a BM type rotary viscometer at 25°C (the same applies hereinafter).

One kind of the component (A1) may be used alone, or two or more kinds thereof may be used in combination.

Specific examples of the component (A1) include the following, but are not limited thereto. Note that, in the following formulae, Me, Vi, and Ph respectively represent a methyl group, a vinyl group, and a phenyl group. 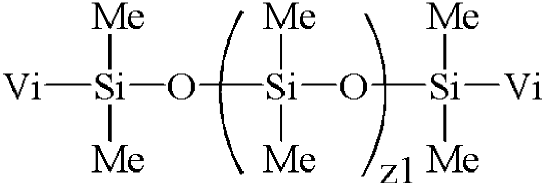 (100≤z1≤5,000) 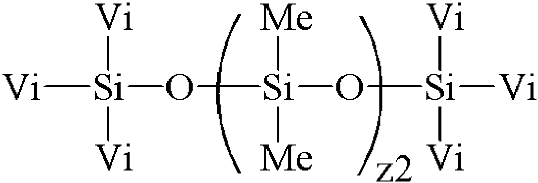 (100≤z2≤15,000) 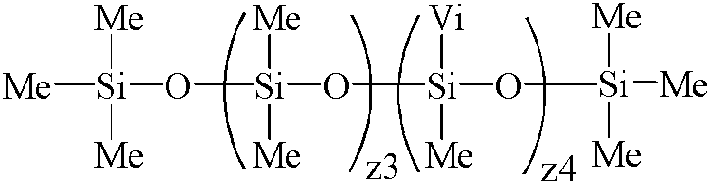 (100≤z3≤19,998, 2≤z4≤2,000, 102≤z3+z4≤20,000) 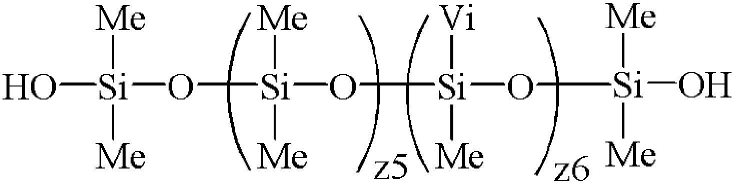 (100≤z5≤19,998, 2≤z6≤2,000, 102≤z5+z6≤20,000) 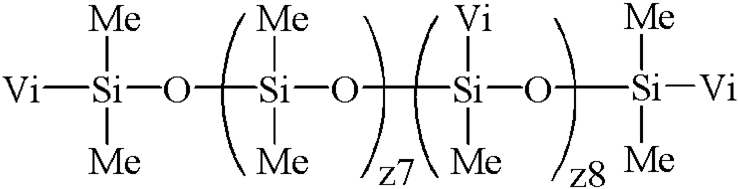 (100≤z7≤19,999, 1≤z8≤2,000, 101≤z7+z8≤20,000) 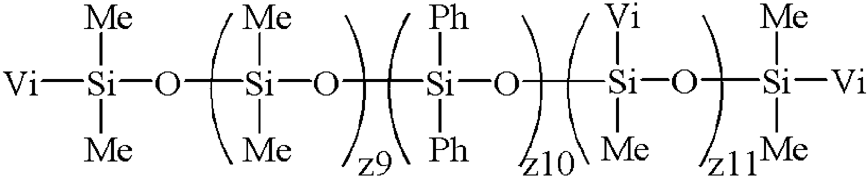 (100≤z9≤19,998, 1≤z10≤6,000, 1≤z11≤3,000, 102≤z9+z10+z11≤20,000)

The component (A2) is a diorganopolysiloxane having a SiOH group at a terminal and no alkenyl group. Examples of the component (A2) include ones shown by the following formula (3). 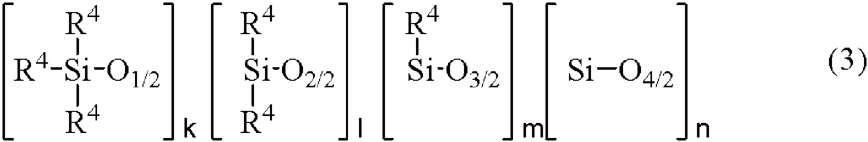 In the formula, each R⁴ independently represents a hydroxyl group, or a monovalent hydrocarbon group having 1 to 10 carbon atoms and no aliphatic unsaturated bond. At least two R⁴'s are hydroxyl groups. "k" represents an integer of 2 or more. "1" represents an integer of 1 or more. "m" represents an integer of 0 or more. "n" represents an integer of 0 or more, with 100≤k+l+m+n≤20,000.

In the formula (3), each R⁴, when not representing a hydroxyl group, is independently a monovalent hydrocarbon group having 1 to 10 carbon atoms, preferably 1 to 8 carbon atoms, and having no aliphatic unsaturated bond. Examples of R⁴ include alkyl groups such as a methyl group, an ethyl group, a propyl group, and a butyl group; cycloalkyl groups such as a cyclohexyl group; and aryl groups such as a phenyl group and a tolyl group. A methyl group or a phenyl group is preferable, and a methyl group is particularly preferable. When a phenyl group is incorporated, the phenyl group content is preferably 0.1 to 30 mol% relative to all the organic groups bonded to the silicon atoms in the diorganopolysiloxane of the formula (3). When the content is 0.1 to 30 mol%, the resulting silicone adhesive layer has preferable adhesion properties (tackiness, adhesive strength), and the suitability of the adhesive for skin application is enhanced.

When the component (A2) is a diorganopolysiloxane shown by the formula (3), the formula (3) is such that "k" is an integer of 2 or more, "1" is an integer of 1 or more, "m" is an integer of 0 or more, and "n" is an integer of 0 or more, with 100≤k+l+m+n≤20,000, preferably 150≤k+l+m+n≤15,000. When k+l+m+n is 100 or more, the viscosity of the composition is not too low and facilitates uniform application. Meanwhile, when k+l+m+n is 20,000 or less, the viscosity of the composition is not excessively high, and the composition is easily stirred and mixed, so that the handleability is favorable.

The diorganopolysiloxane component (A2) may be in a form of oil or raw rubber. When the component (A2) is an oil at 25°C, the viscosity is preferably 300 to 1,000,000 mPa·s, particularly preferably 3,000 to 80,000 mPa·s. When the viscosity is at the lower limit value or more, it is easy to apply the composition uniformly. When the viscosity is at the upper limit value or less, the adhesive composition does not have too high viscosity, and stirring is easily performed when the composition is prepared.

Note that the SiOH group amount of the component (A2) is preferably 0.002 to 0.45 mass%, particularly preferably 0.005 to 0.4 mass%, of the component (A2).

One kind of the component (A2) may be used alone, or two or more kinds thereof may be used in combination.

Specific examples of the component (A2) include the following, but are not limited thereto. Note that, in the following formulae, Me and Ph respectively represent a methyl group and a phenyl group. 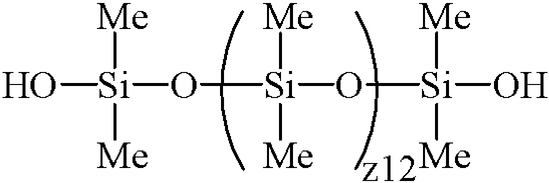 (100≤z12≤5,000) 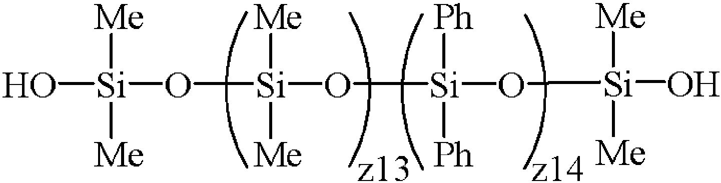 (100≤z13≤19,999, 1≤z14≤2,000, 101≤z13+z14≤20,000)

### <Component (B)>

The component (B) is an organopolysiloxane containing an R¹₃SiO_{0.5} unit (R¹'s each independently represent a monovalent hydrocarbon group having 1 to 10 carbon atoms), a SiO₂ unit, and a siloxane unit having a silicon atom-bonded hydroxyl group and/or a siloxane unit having a silicon atom-bonded alkoxy group with 1 to 6 carbon atoms. The molar ratio of R¹₃SiO_{0.5} unit/SiO₂ unit is 0.5 to 1.5, preferably 0.6 to 1.3. If the molar ratio is less than 0.5, the resulting silicone adhesive layer has low adhesive strength or tackiness. If the molar ratio exceeds 1.5, the resulting silicone adhesive layer has low adhesive strength and holding power.

R¹ is a monovalent hydrocarbon group having 1 to 10 carbon atoms, preferably 1 to 8 carbon atoms. Specific examples thereof include alkyl groups such as a methyl group, an ethyl group, a propyl group, and a butyl group; cycloalkyl groups such as a cyclohexyl group; aryl groups such as a phenyl group; and alkenyl groups such as a vinyl group, an allyl group, and a hexenyl group. A methyl group is preferable. In addition, examples of the alkoxy group having 1 to 6 carbon atoms include a methoxy group, an ethoxy group, a propoxy group, etc.

Additionally, although an R¹₃SiO_{0.5} unit and a SiO₂ unit are essential in the component (B), the component (B) can also contain any one or both of an R¹SiO_{1.5} unit and an R¹₂SiO unit (R¹'s are as defined above) if necessary. Note that, in the component (B), a total content of the R¹₃SiO_{0.5} unit and the SiO₂ unit is not particularly limited, but is preferably 80 to 100 mol%, more preferably 90 to 100 mol%, of all the siloxane units in the component (B).

The component (B) contains a siloxane unit having a silicon atom-bonded hydroxyl group (silanol group-containing unit) and/or a siloxane unit having a silicon atom-bonded alkoxy group with 1 to 6 carbon atoms (alkoxy group-containing unit). Of these, the silanol group-containing unit is preferably in such an amount that the hydroxyl group-content thereof is 0.1 to 5 mass%, preferably 0.2 to 4 mass%, of the component (B). When the hydroxyl group-content is 5 mass% or less, the tackiness and curability of the resulting silicone adhesive layer are not lowered. When the hydroxyl group-content is 0.1 mass% or more, condensation reactions among molecules of the component (B), between the components (A) and (B), or between the components (B) and (C) during curing to be described later proceed sufficiently. Consequently, appropriate adhesive strength is achieved. Further, the alkoxy group-containing unit is preferably in such an amount that the alkoxy group-content thereof is 10 mass% or less, preferably 8 mass% or less, of the component (B). When the alkoxy group-content is 10 mass% or less, the tackiness and curability of the resulting silicone adhesive layer are not lowered. Further, a total amount of the silanol group-containing unit and the alkoxy group-containing unit is such that a total of the hydroxyl group-content and alkoxy group-content described above is 0.1 to 12 mass%, particularly preferably 0.2 to 10 mass%, of the component (B). Note that examples of the silanol group-containing unit include R¹₂(HO)SiO_{0.5} unit, R¹(HO)₂SiO_{0.5} unit, R¹(HO)SiO unit, (HO)SiO_{1.5} unit, etc. Further, examples of the alkoxy group-containing unit include R¹₂(R'O)SiO_{0.5} unit, R¹(R'O)₂SiO_{0.5} unit, R¹(R'O)SiO unit, (R'O)SiO_{1.5} unit, etc. (here, each R'O independently represents an alkoxy group having 1 to 6 carbon atoms, and examples thereof include a methoxy group, an ethoxy group, a propoxy group, etc.)

The component (B) has a weight-average molecular weight of preferably 500 to 10,000, more preferably 1,000 to 8,000. The weight-average molecular weight can be normally determined as, for example, weight-average molecular weight in terms of polystyrene by gel permeation chromatography (GPC) analysis using toluene as a developing solvent (the same applies hereinafter).

Additionally, one kind of the component (B) may be used alone, or two or more kinds thereof may be used in combination.

### <Mixture of Components (A) and (B)>

The inventive addition-curable silicone adhesive composition for skin application can contain a mixture of the components (A) and (B) as the component (X). The mixture of the components (A) and (B) can be obtained by simply mixing the components (A1), (A2), and (B). The means for obtaining the mixture is not particularly limited.

A blend ratio of the components (A) and (B), which is expressed by (A)/(B), is 90/10 to 55/45, preferably 80/20 to 50/50, more preferably 75/25 to 40/60, in term of mass. If the blend ratio is lower than 55/45, adhesive strength upon re-attachment is lowered. Meanwhile, if the blend ratio exceeds 90/10, the resulting adhesive layer has insufficient adhesive strength.

### <Condensation Reaction Product of Components (A) and (B)>

The inventive addition-curable silicone adhesive composition for skin application can contain a condensation reaction product of the components (A) and (B) as the component (X). The condensation reaction product is not particularly limited, as long as it is a product of condensation reaction between the components (A) and (B). The condensation reaction product may contain a partially condensed reaction product between the components (A) and (B) in some cases, and may contain an excessive component of either the component (A) or (B). Moreover, the condensation reaction product may contain unreacted materials of the components (A) and (B). The component (X) is preferably a condensation reaction product among the components (A1), (A2), and (B), or a mixture of the component (A1) with a condensation reaction product between the components (A2) and (B). In this case also, the condensation reaction product may contain a partially condensed reaction product from the component (B) and the component (A1) and/or the component (A2), and may contain unreacted materials of the components (A1), (A2), and (B). For example, when the component (A1) has a functional group capable of condensation reaction, the component (X) may be a condensation reaction product among the components (A1), (A2), and (B); when the component (A1) does not have such a functional group, the component (X) may be a mixture of the component (A1) with a condensation reaction product between the components (A2) and (B). Note that, hereinafter, "a condensation reaction product among the components (A1), (A2), and (B), or a mixture of the component (A1) with a condensation reaction product between the components (A2) and (B)" is also referred to as "component (AB)". The blend ratio of the components (A) and (B), and the mass ratio of (A1)/(A2) in the component (AB) are the same as those in the mixture of the components (A) and (B) .

The condensation reaction between the components (A) and (B) can take place at once or successively. The condensation reaction product may be a condensation reaction product obtained by subjecting all of the component (A1), hydroxyl groups of the component (A2), and alkoxy or hydroxyl groups of the component (B) to hydrolysis condensation reaction or condensation reaction, or may be a mixture of the component (A1) with a condensate obtained by subjecting the components (A2) and (B) to hydrolysis condensation reaction or condensation reaction. Particularly, it is more preferable to use a condensation reaction product obtained by subjecting all the components (A1), (A2), and (B) to condensation reaction in advance. To perform the condensation reaction, a mixture of the components (A1), (A2), and (B) dissolved in a solvent such as toluene is allowed to react by using an alkaline catalyst at room temperature (25°C) or react by heating under reflux, and neutralized as necessary. In this case, the reaction is preferably performed so that the condensation reaction product can have a SiOH group (silanol group) amount of 0.02 to 4.1 mass%, particularly preferably 0.05 to 3.5 mass%.

Here, examples of the alkaline catalyst used in the reaction include metal hydroxides such as lithium hydroxide, sodium hydroxide, potassium hydroxide, and calcium hydroxide; carbonates such as sodium carbonate and potassium carbonate; bicarbonates such as sodium bicarbonate and potassium bicarbonate; metal alkoxides such as sodium methoxide and potassium butoxide; organometallics such as butyllithium; potassium silanolate; nitrogen compounds such as ammonia gas, ammonia water, methylamine, trimethylamine, and triethylamine; etc. Ammonia gas or ammonia water is preferable.

The temperature of the condensation reaction can be 10 to 150°C. Normally, the reaction may be performed from room temperature (25°C) to the reflux temperature of the organic solvent. The reaction time is not particularly limited, and may be 0.5 to 20 hours, preferably 1 to 16 hours.

Further, after completion of the reaction, a neutralizing agent for neutralizing the alkaline catalyst may be added as necessary. Examples of the neutralizing agent include acid gases such as hydrogen chloride and carbon dioxide; organic acids such as acetic acid, octylic acid, and citric acid; mineral acids such as hydrochloric acid, sulfuric acid, and phosphoric acid; etc. When ammonia gas, ammonia water, or low-boiling-point amine compound is used as the alkaline catalyst, the alkaline catalyst may be distilled off by passing an inert gas such as nitrogen therethrough.

### <Component (C)>

The component (C) is an organohydrogenpolysiloxane containing three or more Si-H groups per molecule. One kind of the component (C) can be used alone, or two or more kinds thereof can be used in appropriate combination. The number of Si-H groups per molecule is 3 or more, preferably 5 to 1,000, more preferably 8 to 500. The organohydrogenpolysiloxane component (C) may be linear, branched, or cyclic. The viscosity of this organohydrogenpolysiloxane at 25°C is preferably 1 to 5,000 mPa·s, further preferably 2 to 3,000 mPa·s.

Examples of the organohydrogenpolysiloxane as the component (C) include ones shown by the following formula (1). 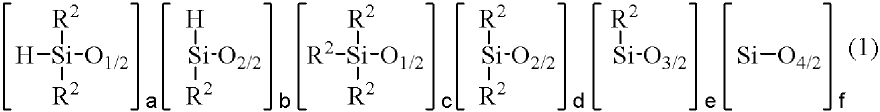 In the formula, each R² is identical to or different from one another and represents a monovalent hydrocarbon group having 1 to 10 carbon atoms and no aliphatic unsaturated bond. "a" represents an integer of 0 or more, "b" represents an integer of 1 or more, "c" represents an integer of 0 or more, "d" represents an integer of 0 or more, "e" represents an integer of 0 or more, and "f" represents an integer of 0 or more, with a+b≥3 and 3≤a+b+c+d+e+f≤1,000.

Here, R²'s are identical or different monovalent hydrocarbon groups each having 1 to 10 carbon atoms, preferably 1 to 8 carbon atoms, but having no aliphatic unsaturated bond. Examples of R² include alkyl groups such as a methyl group, an ethyl group, a propyl group, and a butyl group; cycloalkyl groups such as a cyclohexyl group; aryl groups such as a phenyl group and a tolyl group; etc. Further, some or all of hydrogen atoms bonded to carbon atoms of these groups may be substituted with a halogen atom or another group. Examples of such a substituent include a trifluoromethyl group, a 3,3,3-trifluoropropyl group, etc. Particularly, the substituent is preferably a saturated aliphatic group or an aromatic group, more preferably a methyl group or a phenyl group. A methyl group is particularly preferable.

"a" represents an integer of 0 or more, "b" represents an integer of 1 or more, "c" represents an integer of 0 or more, "d" represents an integer of 0 or more, "e" represents an integer of 0 or more, and "f" represents an integer of 0 or more, with a+b≥3 and 3≤a+b+c+d+e+f≤1,000, preferably 5≤a+b+c+d+e+f≤1,000.

Specific examples of the component (C) include the following, but are not limited thereto. Note that, in the following formulae, Me and Ph respectively represent a methyl group and a phenyl group. 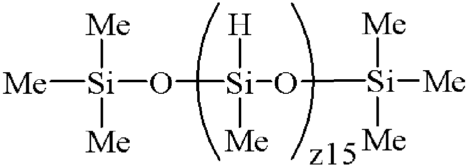 (3≤z15≤1,000) 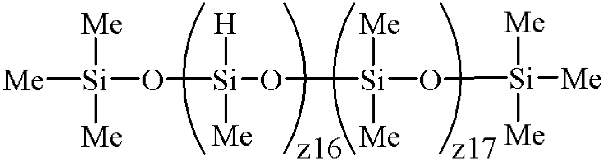 (3≤z16≤999, 1≤z17≤997, 4≤z16+z17≤1,000) 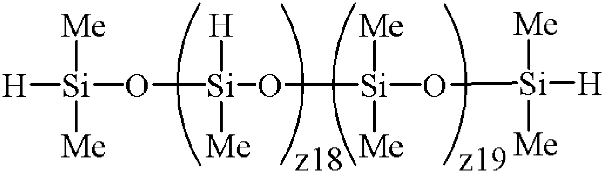 (1≤z18≤999, 1≤z19≤999, 2≤z18+z19≤1,000) 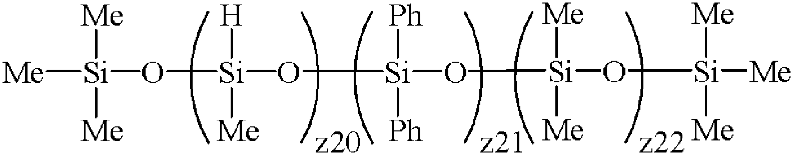 (3≤z20≤998, 1≤z21≤996, 1≤z22≤996, 5≤z20+z21+z22≤1,000) 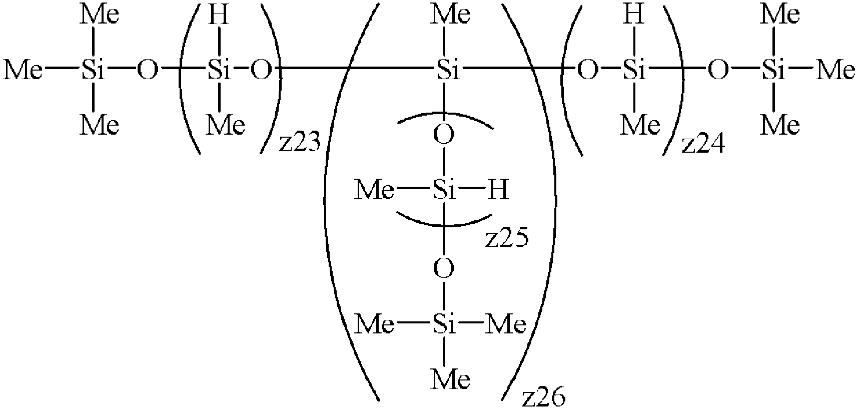 (0≤z23≤200, 0≤z24≤200, 0≤z25≤200, 1≤z26≤50, 3≤z23+z24+(z25×z26)≤1,000)

The component (C) is blended in such an amount that the molar ratio of the Si-H groups in the component (C) to the alkenyl groups in the component (A) (the number of Si-H groups in the component (C)/the number of alkenyl groups in the component (A)) is 0.2 to 15, more preferably 0.5 to 8.5, and further preferably 0.8 to 7.0. If the amount is such that this molar ratio is less than 0.2, the crosslinking density is lowered, so that the cohesion and the holding power may be lowered. Meanwhile, if the mole ratio exceeds 15, the crosslinking density becomes too high, and suitable adhesive strength and tackiness relative to skin are not obtained in some cases.

In comparison with acrylic or rubber-based adhesives, silicone adhesives are characterized by less removal of body hair and the stratum corneum of skin upon peeling, but have a problem that the adhesive strength is decreased when the peeled silicone adhesive is attached again. This is conceivably because the adhesive surface is contaminated with keratin or body hair, and the adhesion-inhibiting component remains between the adhesive surface and skin when the adhesive is re-attached, thereby lowering the adhesive strength.

Moreover, a cause of degrading the reworkability of an adhesive obtained from typical addition reaction-curable silicone adhesive composition is presumably influenced by the hardness of the adhesive. If an adhesive layer is soft, the adhesive layer deforms when the adhesive tape is peeled from skin, thereby suppressing the lifting of the skin. As a result, the adhesive surface is not contaminated with keratin or body hair, and the adhesive strength is not decreased when the adhesive tape is attached again. Meanwhile, if an adhesive layer is hard, the skin is lifted when the adhesive tape is peeled from skin. This causes stronger skin irritation, and keratin and body hair are removed to contaminate the adhesive surface. In the present invention, the ratio between the components (A) and (B) and the ratio between the components (A1) and (A2) are adjusted to obtain a soft silicone adhesive having cohesion and reworkability. Further, since this adhesive is soft, the adhesive easily adapts to uneven structure on skin. Thus, the wettability to skin is excellent.

In the inventive silicone adhesive composition, a molar ratio between a sum of SiH groups and a sum of SiOH groups in the composition (e.g., all the SiOH groups in the components (A) and (B)) is preferably 0.02 to 200,000, particularly preferably 0.1 to 100,000 (SiH groups/SiOH groups). Note that when a condensation reaction product is obtained by subjecting both the components (A) and (B) to condensation reaction in advance, this molar ratio (SiH groups/SiOH groups) between the sums of SiH groups and SiOH groups in the condensation reaction product is preferably 0.05 to 200,000, particularly preferably 0.1 to 100,000.

### <Component (D)>

The component (D) is a platinum group metal-based catalyst. One kind of the component (D) can be used alone, or two or more kinds thereof can be used in appropriate combination. Examples of the platinum group metal-based catalyst include platinum-based catalysts such as chloroplatinic acid, an alcohol solution of chloroplatinic acid, reaction product of chloroplatinic acid with alcohol, reaction product of chloroplatinic acid with olefin compound, reaction product of chloroplatinic acid with vinyl group-containing siloxane, and platinum complex with vinyl group-containing siloxane. Other examples include catalysts containing metals such as ruthenium, rhodium, palladium, and iridium. Above all, platinum-based catalysts are preferable in the present invention.

The component (D) is added in an amount of 1 to 5,000 ppm, preferably 5 to 500 ppm, more preferably 10 to 200 ppm, in terms of platinum group metal relative to the total of the components (A) and (B) based on mass. If the amount is less than 1 ppm, the curability, crosslinking density, and holding power are lowered. If the amount exceeds 5,000 ppm, the available time of the treatment bath is shortened.

Generally, it is not usual to mix silicone adhesives with such catalyst component (D). In the present invention, the component (D) can be used in uniformly mixed state before actual use. It is desirable to add the component (D) immediately before use in view of pot life.

### <Component (E)>

The component (E) is an aliphatic hydrocarbon solvent, but not an aromatic hydrocarbon solvent, and is capable of uniformly dissolving the components (A) and (B) described above. Specific examples of the component (E) include aliphatic hydrocarbon solvents such as hexane, heptane, octane, isooctane, decane, cyclohexane, methylcyclohexane, and isoparaffin (with a boiling point ranging from 115 to 138°C); mixed solvents thereof; etc. Aromatic hydrocarbon solvents such as benzene, toluene, xylene, ethylbenzene, and styrene are excluded from the examples. The component (E) is preferably hexane, heptane, octane, and isoparaffin, more preferably heptane and isoparaffin. The amount of the component (E) is not particularly limited, and is preferably 25 to 900 parts by mass, more preferably 50 to 500 parts by mass, further preferably 60 to 300 parts by mass, relative to 100 parts by mass of the total of the components (A) and (B).

### <Component (F)>

The component (F) is an addition reaction inhibitor. One kind of the component (F) can be used alone, or two or more kinds thereof can be used in appropriate combination. The addition reaction inhibitor is an optional component that can be added to prevent thickening or gelling of a treatment solution containing the silicone adhesive composition before heat curing, for example, when the silicone adhesive composition is prepared or when the silicone adhesive composition is applied to a substrate.

Specific examples of the component (F) include:
3-methyl-1-butyn-3-ol,
3-methyl-1-pentyn-3-ol,
3,5-dimethyl-1-hexyn-3-ol,
1-ethynylcyclohexanol,
3-methyl-3-trimethylsiloxy-1-butyne,
3-methyl-3-trimethylsiloxy-1-pentyne,
3,5-dimethyl-3-trimethylsiloxy-1-hexyne,
1-ethynyl-1-trimethylsiloxycyclohexane,
bis(2,2-dimethyl-3-butynoxy)dimethylsilane,
1,3,5,7-tetramethyl-1,3,5,7-tetravinylcyclotetrasiloxane,
1,1,3,3-tetramethyl-1,3-divinyldisiloxane, etc.

The blend amount of the component (F) is in a range of 0 to 8 parts by mass relative to 100 parts by mass of the total of the components (A) and (B). When the component (F) is blended, the amount is preferably in a range of 0.05 to 8 parts by mass, more preferably 0.1 to 5 parts by mass. When the component (F) is blended in an amount of 8 parts by mass or less, the curability of the resulting composition is not lowered.

### <Other Optional Components>

To the inventive silicone adhesive composition, other optional components than the above-described components can be added. It is possible to use, for example, unreactive organopolysiloxanes such as polydimethylsiloxane and polydimethyldiphenylsiloxane; antioxidants such as phenol, quinone, amine, phosphorus, phosphite, sulfur, and thioether types; light stabilizers such as triazole and benzophenone types; flame retardants such as phosphoric acid ester, halogen, phosphorus, and antimony types; antistatic agents such as cationic, anionic, and nonionic activators; solvents for reducing the viscosity during coating; dyes; pigments; etc. Examples of the solvents for reducing the viscosity during coating include aromatic hydrocarbon solvents such as toluene and xylene; aliphatic hydrocarbon solvents such as hexane, heptane, octane, isooctane, decane, cyclohexane, methylcyclohexane, and isoparaffin; hydrocarbon solvents such as industrial gasoline, petroleum benzene, and solvent naphtha; ketone solvents such as acetone, methyl ethyl ketone, 2-pentanone, 3-pentanone, 2-hexanone, 2-heptanone, 4-heptanone, methyl isobutyl ketone, diisobutyl ketone, acetonylacetone, and cyclohexanone; ester solvents such as ethyl acetate, propyl acetate, isopropyl acetate, butyl acetate, and isobutyl acetate; ether solvents such as diethyl ether, dipropyl ether, diisopropyl ether, dibutyl ether, 1,2-dimethoxyethane, and 1,4-dioxane; solvents having ester and ether moieties such as 2-methoxyethyl acetate, 2-ethoxyethyl acetate, propylene glycol monomethyl ether acetate, and 2-butoxyethyl acetate; siloxane-based solvents such as hexamethyldisiloxane, octamethyltrisiloxane, octamethylcyclotetrasiloxane, decamethylcyclopentasiloxane, tris(trimethylsiloxy)methylsilane and tetrakis(trimethylsiloxy)silane; and mixed solvents thereof. One of these optional components can be used alone, or two or more kinds thereof can be used in appropriate combination. Note that the aforementioned solvents as optional components may be added to the silicone adhesive composition separately from the component (E), so that the composition can be adjusted to have preferable viscosity and so forth upon application.

### <Composition Production Method>

The inventive silicone adhesive composition can be obtained by mixing the components (A) to (D), and as necessary (E), (F), and other component(s). The order of mixing the components is not particularly limited. Moreover, as described above, the component (AB) obtained by condensation reaction between the components (A) and (B) can be used instead of the components (A) and (B). The component (AB) can be obtained as follows, for example. A solution containing the components (A1), (A2), and (B), as well as if necessary the component (E) (solvent) and an alkaline catalyst (such as ammonia water), is stirred at room temperature to obtain a condensation product between the component (A2) and the component (B). Further, the condensation product is heated with refluxing to thus obtain the reaction product (a mixture of the component (A1) with the condensation product between the component (A2) and the component (B)).

Then, when necessary, the component (F) and the component (E) are added to the components (A) and (B) or the component (AB), and mixed to synthesize a silicone adhesive base composition. This base composition is mixed with the component (C) and the component (D). Thus, a silicone adhesive composition is prepared.

### <Usage of Composition>

The inventive addition reaction-curable silicone adhesive composition for skin application produced as described can be applied to various substrates and cured under predetermined conditions. In this manner, a silicone adhesive layer is prepared.

### <Adhesive Tape for Skin Application>

For example, the inventive addition reaction-curable silicone adhesive composition for skin application can be suitably used for an adhesive tape for skin application. The adhesive tape for skin application has: a substrate; and an adhesive layer formed of a cured product of the addition reaction-curable silicone adhesive composition for skin application on at least one side of the substrate.

The substrate is selected from papers, plastic films made of plastics, unwoven fabrics, laminate films, glasses, metals, clothes, etc. Examples of the papers include wood-free paper, coated paper, art paper, glassine paper, polyethylene laminate paper, kraft paper, *Washi* (traditional Japanese paper), synthetic paper, etc. Examples of the plastic films include polyethylene film, polypropylene film, polyester film, polyimide film, polyamide film, polyurethane film, polyurea film, polyvinyl chloride film, polyvinylidene chloride film, polyvinyl alcohol film, polycarbonate film, polytetrafluoroethylene film, polystyrene film, ethylene-vinyl acetate copolymer film, ethylene-vinyl alcohol copolymer film, triacetyl cellulose film, polyether ether ketone film, polyphenylene sulfide film, etc. Examples of the unwoven fabrics include synthetic fiber unwoven fabrics, natural fiber unwoven fabrics, etc. Examples of the laminate films include laminate of unwoven fabric and plastic film, laminate of paper and plastic film, etc. The thickness, type, and so forth of the glasses are not particularly limited, and the glasses may be chemically reinforced, for example. It is also possible to employ glass fibers. The glass fibers may be used alone, or a composite with another resin may be used. Examples of the metals include aluminum foil, copper foil, gold foil, silver foil, nickel foil, etc.

To further enhance the adhesiveness between these substrates and the silicone adhesive layer, the substrate to be used may be subjected to primer treatment, corona treatment, etching treatment, or plasma treatment.

As the application/coating method, known application means can be employed for the application. Examples thereof include comma coaters, lip coaters, roll coaters, die coaters, knife coaters, blade coaters, rod coaters, bar coaters, kiss coaters, gravure coaters, screen coating, dip coating, cast coating, etc. In the application, appropriate dilution may be performed with a solvent described above as the optional component. In this case, the content of the silicone adhesive composition in the solution can be 20 to 60 mass%.

The application amount is appropriately set according to the usage. Normally, the amount is preferably such that the silicone adhesive layer after curing has a thickness of 2 to 2,000 µm, particularly preferably 3 to 1,000 µm.

The conditions for curing the addition reaction-curable silicone adhesive composition may be 70 to 250°C and 10 seconds to 10 minutes, but are not limited thereto.

The inventive adhesive tape for skin application may be produced by directly applying the inventive addition reaction-curable silicone adhesive composition to a substrate as described above, and then curing the composition to form a silicone adhesive layer. Alternatively, the inventive adhesive tape for skin application may be produced by a transfer method in which the composition is applied to a release film or release paper having been subjected to release coating, and cured to form a silicone adhesive layer, and then this silicone adhesive layer is attached to the substrate.

### <Use of Adhesive Tape for Skin Application>

In use, the inventive adhesive tape for skin application can be attached to skin. This adhesive tape for skin application has suitable adhesive strength to human skin, so that the substrate can be securely fixed to skin. Further, a decrease in the adhesive strength upon re-attachment is suppressed. The reworkability and durability are excellent, and the inventive adhesive tape for skin application is gently usable with respect to human skin.

### EXAMPLE

Hereinafter, the present invention will be specifically described by showing Synthesis Examples, Examples, and Comparative Examples. However, the present invention is not limited to the following Examples. Note that, in the examples, viscosity values are those at 25°C, part(s) mean "part(s) by mass," and characteristic values are expressed by values measured in test methods to be described later. Moreover, in the examples, Me represents a methyl group, and Vi represents a vinyl group.

### <Raw Materials Used>

### Component (A)

### (A-1)

Dimethylpolysiloxane shown by the following formula. The viscosity was 4,000 mPa·s when this component was dissolved in toluene to make the concentration of 30 mass%. The two terminals of the molecular chain were blocked by vinyl groups. The vinyl group amount was 0.002 mol/100 g. 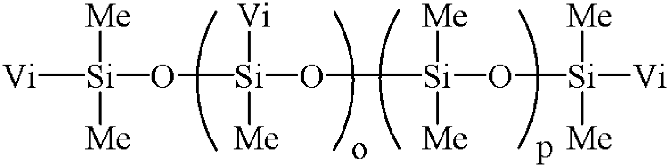 (Here, "o" and "p" represent the numbers satisfying the viscosity)

### (A-2)

Dimethylpolysiloxane shown by the following formula. The viscosity was 1,400 mPa·s when this component was dissolved in toluene to make the concentration of 30 mass%. The two terminals of the molecular chain were blocked by hydroxyl groups. 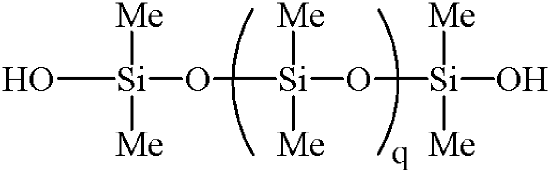 (Here, "q" represents the number satisfying the viscosity)

### Component (B)

### (B-1)

Polysiloxane consisting of an Me₃SiO_{0.5} unit, a SiO₂ unit, and a siloxane unit having a silicon atom-bonded hydroxyl group (Me₃SiO_{0.5} unit/SiO₂ unit (molar ratio)=0.80, the content of the silicon atom-bonded hydroxyl group: 1.2 mass%, weight-average molecular weight: 4,000)

### Component (C)

Methylhydrogenpolysiloxane shown by the following formula

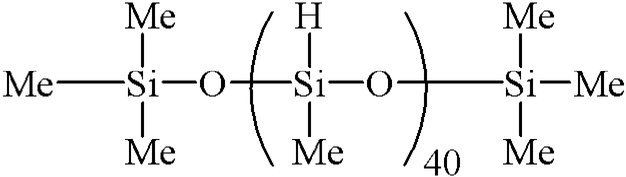

### Component (D)

Complex of platinum and vinyl group-containing siloxane (platinum content: 0.5%)
Component (E)
n-Heptane
Component (F)
Ethynylcyclohexanol

### [Synthesis Example 1]

A solution made up of (A-1) (64.0 parts) as the component (A1), (A-2) (16.0 parts) as the component (A2), (B-1) (20.0 parts) as the component (B), n-heptane (66.1 parts), and ammonia water (0.5 parts) was stirred at room temperature (25°C, hereinafter the same) for 12 hours. Thereby, a condensation product of the components (A) and (B) was obtained. Note that this (A-1) had SiOH groups remaining at some unblocked terminals and was capable of condensation with (B-1). Hence, (A-1) and (A-2) condensed with (B-1), resulting in the condensation product. Next, the resultant was heated with refluxing at approximately 100°C to 115°C for 6 hours to distill off ammonia and water. After the obtained reaction product was allowed to cool, the component (F) (0.2 parts) was added thereto, and n-heptane was added such that the solid content was approximately 60 mass%. The solution was mixed to synthesize a silicone adhesive base composition A.

### [Synthesis Example 2]

A solution made up of (A-1) (16.0 parts) as the component (A1), (A-2) (64.0 parts) as the component (A2), (B-1) (20.0 parts) as the component (B), n-heptane (66.4 parts), and ammonia water (0.5 parts) was stirred at room temperature for 12 hours. Thereby, a condensation product of the components (A) and (B) was obtained. Next, the resultant was heated with refluxing at approximately 100°C to 115°C for 6 hours to distill off ammonia and water. After the obtained reaction product was allowed to cool, the component (F) (0.2 parts) was added thereto, and n-heptane was added such that the solid content was approximately 60 mass%. The solution was mixed to synthesize a silicone adhesive base composition B.

### [Synthesis Example 3]

A solution made up of (A-1) (48.0 parts) as the component (A1), (A-2) (12.0 parts) as the component (A2), (B-1) (40.0 parts) as the component (B), n-heptane (66.2 parts), and ammonia water (0.5 parts) was stirred at room temperature for 12 hours. Thereby, a condensation product of the components (A) and (B) was obtained. Next, the resultant was heated with refluxing at approximately 100°C to 115°C for 6 hours to distill off ammonia and water. After the obtained reaction product was allowed to cool, the component (F) (0.2 parts) was added thereto, and n-heptane was added such that the solid content was approximately 60 mass%. The solution was mixed to synthesize a silicone adhesive base composition C.

### [Synthesis Example 4]

A solution made up of (A-1) (24.0 parts) as the component (A1), (A-2) (36.0 parts) as the component (A2), (B-1) (40.0 parts) as the component (B), n-heptane (66.3 parts), and ammonia water (0.5 parts) was stirred at room temperature for 12 hours. Thereby, a condensation product of the components (A) and (B) was obtained. Next, the resultant was heated with refluxing at approximately 100°C to 115°C for 6 hours to distill off ammonia and water. After the obtained reaction product was allowed to cool, the component (F) (0.2 parts) was added thereto, and n-heptane was added such that the solid content was approximately 60 mass%. The solution was mixed to synthesize a silicone adhesive base composition D.

### [Synthesis Example 5]

A solution made up of (A-1) (38.0 parts) as the component (A1), (A-2) (57.0 parts) as the component (A2), (B-1) (5.0 parts) as the component (B), n-heptane (66.2 parts), and ammonia water (0.5 parts) was stirred at room temperature for 12 hours. Thereby, a condensation product of the components (A) and (B) was obtained. Next, the resultant was heated with refluxing at approximately 100°C to 115°C for 6 hours to distill off ammonia and water. After the obtained reaction product was allowed to cool, the component (F) (0.2 parts) was added thereto, and n-heptane was added such that the solid content was approximately 60 mass%. The solution was mixed to synthesize a silicone adhesive base composition E.

### [Synthesis Example 6]

A solution made up of (A-1) (80.0 parts) as the component (A1), (B-1) (20.0 parts) as the component (B), n-heptane (66.0 parts), and ammonia water (0.5 parts) was stirred at room temperature for 12 hours. Thereby, a condensation product of the components (A) and (B) was obtained. Note that since this (A-1) was capable of condensation with (B-1) as described above, (A-1) condensed with (B-1), resulting in the condensation product. Next, the resultant was heated with refluxing at approximately 100°C to 115°C for 6 hours to distill off ammonia and water. After the obtained reaction product was allowed to cool, the component (F) (0.2 parts) was added thereto, and n-heptane was added such that the solid content was approximately 60 mass%. The solution was mixed to synthesize a silicone adhesive base composition F.

### [Synthesis Example 7]

A solution made up of (A-1) (8.0 parts) as the component (A1), (A-2) (72.0 parts) as the component (A2), (B-1) (20.0 parts) as the component (B), n-heptane (66.4 parts), and ammonia water (0.5 parts) was stirred at room temperature for 12 hours. Thereby, a condensation product of the components (A) and (B) was obtained. Next, the resultant was heated with refluxing at approximately 100°C to 115°C for 6 hours to distill off ammonia and water. After the obtained reaction product was allowed to cool, the component (F) (0.2 parts) was added thereto, and n-heptane was added such that the solid content was approximately 60 mass%. The solution was mixed to synthesize a silicone adhesive base composition G.

### [Synthesis Example 8]

A solution made up of (A-1) (60.0 parts) as the component (A1), (B-1) (40.0 parts) as the component (B), n-heptane (66.1 parts), and ammonia water (0.5 parts) was stirred at room temperature for 12 hours. Thereby, a condensation product of the components (A) and (B) was obtained. Next, the resultant was heated with refluxing at approximately 100°C to 115°C for 6 hours to distill off ammonia and water. After the obtained reaction product was allowed to cool, the component (F) (0.2 parts) was added thereto, and n-heptane was added such that the solid content was approximately 60 mass%. The solution was mixed to synthesize a silicone adhesive base composition H.

### [Synthesis Example 9]

A solution made up of (A-1) (6.0 parts) as the component (A1), (A-2) (54.0 parts) as the component (A2), (B-1) (40.0 parts) as the component (B), n-heptane (66.4 parts), and ammonia water (0.5 parts) was stirred at room temperature for 12 hours. Thereby, a condensation product of the components (A) and (B) was obtained. Next, the resultant was heated with refluxing at approximately 100°C to 115°C for 6 hours to distill off ammonia and water. After the obtained reaction product was allowed to cool, the component (F) (0.2 parts) was added thereto, and n-heptane was added such that the solid content was approximately 60 mass%. The solution was mixed to synthesize a silicone adhesive base composition I.

### [Synthesis Example 10]

A solution made up of (A-1) (20.0 parts) as the component (A1), (A-2) (30.0 parts) as the component (A2), (B-1) (50.0 parts) as the component (B), n-heptane (66.3 parts), and ammonia water (0.5 parts) was stirred at room temperature for 12 hours. Thereby, a condensation product of the components (A) and (B) was obtained. Next, the resultant was heated with refluxing at approximately 100°C to 115°C for 6 hours to distill off ammonia and water. After the obtained reaction product was allowed to cool, the component (F) (0.2 parts) was added thereto, and n-heptane was added such that the solid content was approximately 60 mass%. The solution was mixed to synthesize a silicone adhesive base composition J.

### [Synthesis Example 11]

A solution made up of (A-1) (36.0 parts) as the component (A1), (A-2) (54.0 parts) as the component (A2), (B-1) (10.0 parts) as the component (B), n-heptane (66.2 parts), and ammonia water (0.5 parts) was stirred at room temperature for 12 hours. Thereby, a condensation product of the components (A) and (B) was obtained. Next, the resultant was heated with refluxing at approximately 100°C to 115°C for 6 hours to distill off ammonia and water. After the obtained reaction product was allowed to cool, the component (F) (0.2 parts) was added thereto, and n-heptane was added such that the solid content was approximately 60 mass%. The solution was mixed to synthesize a silicone adhesive base composition K.

### [Synthesis Example 12]

A solution made up of (A-1) (72.0 parts) as the component (A1), (A-2) (8.0 parts) as the component (A2), (B-1) (20.0 parts) as the component (B), n-heptane (66.0 parts), and ammonia water (0.5 parts) was stirred at room temperature for 12 hours. Thereby, a condensation product of the components (A) and (B) was obtained. Next, the resultant was heated with refluxing at approximately 100°C to 115°C for 6 hours to distill off ammonia and water. After the obtained reaction product was allowed to cool, the component (F) (0.2 parts) was added thereto, and n-heptane was added such that the solid content was approximately 60 mass%. The solution was mixed to synthesize a silicone adhesive base composition L.

### [Synthesis Example 13]

A solution made up of (A-1) (12.0 parts) as the component (A1), (A-2) (68.0 parts) as the component (A2), (B-1) (20.0 parts) as the component (B), n-heptane (66.0 parts), and ammonia water (0.5 parts) was stirred at room temperature for 12 hours. Thereby, a condensation product of the components (A) and (B) was obtained. Next, the resultant was heated with refluxing at approximately 100°C to 115°C for 6 hours to distill off ammonia and water. After the obtained reaction product was allowed to cool, the component (F) (0.2 parts) was added thereto, and n-heptane was added such that the solid content was approximately 60 mass%. The solution was mixed to synthesize a silicone adhesive base composition M.

### [Synthesis Example 14]

A solution made up of (A-1) (22.0 parts) as the component (A1), (A-2) (33.0 parts) as the component (A2), (B-1) (45.0 parts) as the component (B), n-heptane (66.0 parts), and ammonia water (0.5 parts) was stirred at room temperature for 12 hours. Thereby, a condensation product of the components (A) and (B) was obtained. Next, the resultant was heated with refluxing at approximately 100°C to 115°C for 6 hours to distill off ammonia and water. After the obtained reaction product was allowed to cool, the component (F) (0.2 parts) was added thereto, and n-heptane was added such that the solid content was approximately 60 mass%. The solution was mixed to synthesize a silicone adhesive base composition N.

### <Examples 1 to 10, Comparative Examples 1 to 6>

The silicone adhesive base compositions A to N described above and the component (C) were placed in flasks according to the blend amounts in tables, diluted with 83.4 parts of n-heptane, stirred, and dissolved. To the obtained solutions, 0.8 parts of the component (D) was added, stirred, and mixed to prepare silicone adhesive compositions. From the silicone adhesive compositions, cured products were prepared by a method to be described later. The adhesive strength to skin and the adhesive strength upon re-attachment were measured according to the following measurement methods. The tables show the results.

### [Adhesive Strength to Skin]

Onto a polyethylene terephthalate film having a thickness of 23 µm and a width of 25 mm, a 40 mass% n-heptane solution of each silicone adhesive composition was applied using an applicator in such an amount that the thickness after curing was to be 100 µm. Then, the composition was cured by heating under conditions of 130°C and 1 minute to prepare an adhesive tape. This adhesive tape was attached to a forearm of a test subject and left standing at room temperature for approximately 30 minutes. Subsequently, at 25°C, one end of the adhesive tape was peeled, and thereafter a force (N/25mm) required to peel away the adhesive tape from the forearm at an angle of 90° at a tensile rate of 60 mm/minute was measured using a tensile testing machine.

### [Adhesive Strength to Skin After Re-Attachment]

The adhesive tape peeled in the measurement of the adhesive strength to skin was attached again to the forearm of the test subject, and left standing at room temperature for approximately 30 minutes. Then, at 25°C, one end of the adhesive tape was peeled, and thereafter a force (N/25mm) required to peel away the adhesive tape from the forearm at an angle of 90° at a tensile rate of 60 mm/minute was measured using the tensile testing machine.

### [Reworkability]

In the measurement of the adhesive strength to skin, the reworkability on the forearm of the test subject was evaluated based on the following 3-stage criteria.
A: the decrease percentage of adhesive strength upon re-attachment was less than 15%
B: the decrease percentage of adhesive strength upon re-attachment was 15% or more and less than 30%
C: the decrease percentage of adhesive strength upon re-attachment was 30% or more

### [Evaluation of Peeling from Skin]

In the measurement of the adhesive strength to skin, the pain during the peeling from the forearm of the test subject was evaluated based on the following 3-stage criteria.
A: no pain was felt
B: slight pain was felt
C: great pain was felt

### [Appearance of Peeled Adhesive Tape]

In checking the adhesive strength to skin after re-attachment, the appearance of the adhesive tape peeled after the re-attachment was observed and evaluated based on the following 3-stage criteria.
A: little keratin and body hair were observed
B: keratin and body hair were observed
C: a lot of keratin and body hair were observed

### [Durability of Adhesive Tape]

Onto a polyethylene terephthalate film having a thickness of 23 pm and a width of 25 mm, a 40 mass% n-heptane solution of each silicone adhesive composition was applied using an applicator in such an amount that the thickness after curing was to be 100 pm. Then, the composition was cured by heating under conditions of 130°C and 1 minute to prepare an adhesive tape. This adhesive tape was attached to the forearm of the test subject. The state of the test piece 24 hours after the attachment was observed and evaluated based on the following 3-stage criteria, depending on occurrences of deformation, crease, and peeling of the test piece, and the paste remaining after peeling.
A: hardly occurred
B: somewhat occurred
C: significantly occurred

**[Table 1]**

| | Example | | | | | |
|---|---|---|---|---|---|---|
| | 1 | 2 | 3 | 4 | 5 | 6 |
| Silicone adhesive base composition A [parts] | 166.7 | 83.35 | - | - | - | - |
| Silicone adhesive base composition B [parts] | - | 83.35 | 166.7 | - | - | - |
| Silicone adhesive base composition C [parts] | - | - | - | 166.7 | 83.35 | - |
| Silicone adhesive base composition D [parts] | - | - | - | - | 83.35 | 166.7 |
| Component (C) [parts] | 0.41 | 0.257 | 0.103 | 0.308 | 0.231 | 0.154 |
| Component (A) (parts)/ component (B) (parts) | 80/20 | 80/20 | 80/20 | 60/40 | 60/40 | 60/40 |
| Component (A-1) (parts)/ component (A-2) (parts) | 80/20 | 50/50 | 20/80 | 80/20 | 60/40 | 40/60 |
| SiH in (C)(mol)/ SiVi in (A)(mol) | 5 | 5 | 5 | 5 | 5 | 5 |
| Adhesive strength to skin [N/25mm] | 0.31 | 0.35 | 1.13 | 1.29 | 1.55 | 3.66 |
| Adhesive strength to skin upon re-attachment [N/25mm] | 0.27 | 0.30 | 1.02 | 1.10 | 1.32 | 2.85 |
| Reworkability | A | A | A | A | A | B |
| Evaluation of peeling from skin | A | A | A | A | A | B |
| Appearance of peeled adhesive tape | A | A | A | A | A | A |
| Durability of adhesive tape | A | A | A | A | A | A |

**[Table 2]**

| | Example | | | |
|---|---|---|---|---|
| | 7 | 8 | 9 | 10 |
| Silicone adhesive base composition K [parts] | 166.7 | | | |
| Silicone adhesive base composition L [parts] | | 166.7 | | |
| Silicone adhesive base composition M [parts] | | | 166.7 | |
| Silicone adhesive base composition N [parts] | | | | 166.7 |
| Component (C) [parts] | 0.231 | 0.462 | 0.077 | 0.141 |
| Component (A) (parts)/ component (B) (parts) | 90/10 | 80/20 | 80/20 | 55/45 |
| Component (A-1) (parts)/ component (A-2) (parts) | 40/60 | 90/10 | 15/85 | 40/60 |
| SiH in (C)(mol)/ SiVi in (A)(mol) | 5 | 5 | 5 | 5 |
| Adhesive strength to skin [N/25mm] | 0.38 | 0.34 | 2.27 | 3.85 |
| Adhesive strength to skin upon re-attachment [N/25mm] | 0.33 | 0.31 | 2.03 | 3.10 |
| Reworkability | A | A | A | B |
| Evaluation of peeling from skin | A | A | A | B |
| Appearance of peeled adhesive tape | A | A | A | A |
| Durability of adhesive tape | A | A | A | A |

**[Table 3]**

| | Comparative Example | | | | | |
|---|---|---|---|---|---|---|
| | 1 | 2 | 3 | 4 | 5 | 6 |
| Silicone adhesive base composition E [parts] | 166.7 | - | - | - | - | - |
| Silicone adhesive base composition F [parts] | - | 166.7 | - | - | - | - |
| Silicone adhesive base composition G [parts] | - | - | 166.7 | - | - | - |
| Silicone adhesive base composition H [parts] | - | - | - | 166.7 | - | - |
| Silicone adhesive base composition I [parts] | - | - | - | - | 166.7 | - |
| Silicone adhesive base composition J [parts] | - | - | - | - | - | 166.7 |
| Component (C) [parts] | 0.244 | 0.513 | 0.051 | 0.385 | 0.038 | 0.128 |
| Component (A) (parts)/ component (B) (parts) | 95/5 | 80/20 | 80/20 | 60/40 | 60/40 | 50/50 |
| Component (A-1) (parts)/ component (A-2) (parts) | 40/60 | 100/0 | 10/90 | 100/0 | 10/90 | 40/60 |
| SiH in (C)(mol)/ SiVi in (A)(mol) | 5 | 5 | 5 | 5 | 5 | 5 |
| Adhesive strength to skin [N/25mm] | fell | 0.23 | 5.57 | 0.77 | 6.55 | 5.89 |
| Adhesive strength to skin upon re-attachment [N/25mm] | fell | 0.16 | 4.25 | 0.51 | 4.35 | 3.15 |
| Reworkability | - | C | B | C | C | C |
| Evaluation of peeling from skin | - | A | C | A | C | C |
| Appearance of peeled adhesive tape | - | A | C | A | C | C |
| Durability of adhesive tape | C | C | A | A | A | A |

As shown in Tables 1 to 3, the silicone adhesive compositions of Examples 1 to 10 (the present invention) apparently resulted in adhesive layers that have suitable adhesive strength to human skin, suppress a decrease in the adhesive strength when re-attached, and exhibit excellent reworkability and durability. In contrast, Comparative Examples 1 to 6, which are outside the scope of the present invention, apparently cannot satisfy all of suitable adhesive strength, adhesive strength upon re-attachment, reworkability, and durability simultaneously. Therefore, the inventive silicone adhesive composition results in an adhesive layer that demonstrates excellent performances as described above, and adhesives tapes for skin application including such adhesive layers are highly valuable.

It should be noted that the present invention is not limited to the above-described embodiments. The embodiments are just examples, and any examples that have substantially the same feature and demonstrate the same functions and effects as those in the technical concept disclosed in claims of the present invention are included in the technical scope of the present invention.

## Claims

1. An addition reaction-curable silicone adhesive composition for skin application, comprising the following components (X), (C), and (D):
(X) 100 parts by mass of a mixture of the following component (A) with the following component (B), or a condensation reaction product between the following component (A) and the following component (B),
(A) 55 to 90 parts by mass of a diorganopolysiloxane consisting of the following components (A1) and (A2), wherein a mass ratio of (A1)/(A2) is within a range from 90/10 to 15/85,
(A1) a diorganopolysiloxane having two or more alkenyl groups per molecule and an alkenyl group-content of at least 0.0005 mol/100 g and less than 0.15 mol/100 g, and wherein the molecular chain terminals are not completely blocked in that some SiOH groups are left remaining, and
(A2) a diorganopolysiloxane having a SiOH group at a terminal and no alkenyl group, and
(B) 10 to 45 parts by mass of an organopolysiloxane, provided that a total of the components (A) and (B) is 100 parts by mass, wherein the organopolysiloxane contains an R¹₃SiO_{0.5} unit, a SiOz unit, and a siloxane unit having a silicon atom-bonded hydroxyl group and/or a siloxane unit having a silicon atom-bonded alkoxy group with 1 to 6 carbon atoms, each R¹ independently represents a monovalent hydrocarbon group having 1 to 10 carbon atoms, and a molar ratio of the R¹₃SiO_{0.5} unit/the SiOz unit is 0.5 to 1.5;
(C) an organohydrogenpolysiloxane containing three or more SiH groups per molecule, wherein the organohydrogenpolysiloxane is in such an amount that a molar ratio of the SiH groups in the component (C) to the alkenyl groups in the component (A) is 0.2 to 15; and
(D) a platinum group metal-based catalyst in an amount of 1 to 5,000 ppm in terms of platinum group metal relative to the total amount of the components (A) and (B) based on mass.

2. The addition reaction-curable silicone adhesive composition for skin application according to claim 1, further comprising the following component (E) in an amount of 25 to 900 parts by mass relative to 100 parts by mass of the total of the components (A) and (B),
(E) at least one aliphatic hydrocarbon solvent selected from the group consisting of hexane, heptane, isooctane, octane, decane, cyclohexane, methylcyclohexane, and isoparaffin with a boiling point ranging from 115°C to 138°C.

3. The addition reaction-curable silicone adhesive composition for skin application according to claim 1 or 2, wherein the component (C) is an organohydrogenpolysiloxane shown by the following formula (1), 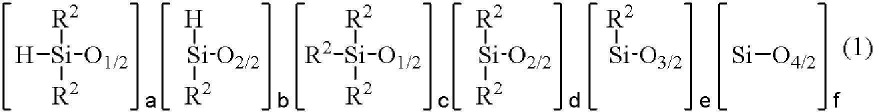 wherein each R² is identical to or different from one another and represents a monovalent hydrocarbon group having 1 to 10 carbon atoms and no aliphatic unsaturated bond, "a" represents an integer of 0 or more, "b" represents an integer of 1 or more, "c" represents an integer of 0 or more, "d" represents an integer of 0 or more, "e" represents an integer of 0 or more, and "f" represents an integer of 0 or more, with a+b≥3 and 3≤a+b+c+d+e+f≤1,000.

4. The addition reaction-curable silicone adhesive composition for skin application according to any one of claims 1 to 3, wherein the component (A) is in such an amount that the mass ratio of (A1)/(A2) is within a range from 75/25 to 25/75.

5. The addition reaction-curable silicone adhesive composition for skin application according to any one of claims 1 to 4, wherein the component (X) is a condensation reaction product among the components (A1), (A2), and (B), or a mixture of the component (A1) with a condensation reaction product between the components (A2) and (B).

6. The addition reaction-curable silicone adhesive composition for skin application according to any one of claims 1 to 5, further comprising an addition reaction inhibitor as a component (F) in an amount of 0.05 to 8 parts by mass relative to 100 parts by mass of the total of the components (A) and (B).

7. An adhesive tape for skin application, comprising:
a substrate; and
a cured product layer laminated on at least one side of the substrate, wherein
the cured product layer is formed of a cured product of the composition according to any one of claims 1 to 6.

8. A use of the adhesive tape for skin application according to claim 7, comprising attaching the adhesive tape for skin application to skin.

## Patentansprüche

1. Additionsreaktionshärtbare Silikonklebstoffzusammensetzung zur Hautanwendung, umfassend die folgenden Komponenten (X), (C) und (D):
(X) 100 Masseteile eines Gemischs der folgenden Komponente (A) mit der folgenden Komponente (B), oder ein Kondensationsreaktionsprodukt zwischen der folgenden Komponente (A) und der folgenden Komponente (B),
(A) 55 bis 90 Massenteile eines Diorganopolysiloxans, bestehend aus den folgenden Komponenten (A1) und (A2), wobei das Massenverhältnis von (A1)/(A2) in einem Bereich von 90/10 bis 15/85 liegt,
(A1) ein Diorganopolysiloxan mit zwei oder mehr Alkenylgruppen pro Molekül und einem Alkenylgruppen-Gehalt von zumindest 0,0005 mol/100 g und weniger als 0,15 mol/100 g, und wobei die Molekülkettenenden nicht vollständig blockiert sind, so dass einige SiOH-Gruppen übrig bleiben, und
(A2) ein Diorganopolysiloxan mit einer SiOH-Gruppe an einem Ende und keiner Alkenylgruppe, und
(B) 10 bis 45 Massenteile eines Organopolysiloxans, mit der Maßgabe, dass die Summe der Komponenten (A) und (B) 100 Massenteile beträgt, wobei das Organopolysiloxan eine R¹₃SiO_{0.5}-Einheit, eine SiO₂-Einheit und eine Siloxan-Einheit mit einer an ein Siliciumatom gebundenen Hydroxylgruppe und/oder eine Siloxan-Einheit mit einer an ein Siliciumatom gebundenen Alkoxygruppe mit 1 bis 6 Kohlenstoffatomen enthält, wobei jedes R¹ unabhängig voneinander eine einwertige Kohlenwasserstoffgruppe mit 1 bis 10 Kohlenstoffatomen darstellt und ein molares Verhältnis der R¹₃SiO_{0.5}-Einheit zur SiO₂-Einheit 0,5 bis 1,5 beträgt;
(C) ein Organohydrogenpolysiloxan, welches drei oder mehr SiH-Gruppen pro Molekül enthält, wobei das Organohydrogenpolysiloxan in einer solchen Menge vorliegt, dass ein molares Verhältnis der SiH-Gruppen in der Komponente (C) zu den Alkenylgruppen in der Komponente (A) 0,2 bis 15 beträgt; und
(D) ein auf Platingruppenmetall basierender Katalysator in einer Menge von 1 bis 5000 ppm, bezogen auf das Platingruppenmetall relativ zu der Gesamtmenge der Komponenten (A) und (B) basierend auf der Masse.

2. Die additionsreaktionshärtbare Silikonklebstoffzusammensetzung zur Hautanwendung gemäß Anspruch 1, ferner umfassend die folgende Komponente (E) in einer Menge von 25 bis 900 Masseteilen relativ zu 100 Masseteilen der Summe der Komponenten (A) und (B),
(E) zumindest ein aliphatisches Kohlenwasserstoff-Lösungsmittel, ausgewählt aus der Gruppe bestehend aus Hexan, Heptan, Isooctan, Octan, Decan, Cyclohexan, Methylcyclohexan und Isoparaffin mit einem Siedepunkt im Bereich von 115°C bis 138°C.

3. Die additionsreaktionshärtbare Silikonklebstoffzusammensetzung zur Hautanwendung gemäß Anspruch 1 oder 2, wobei die Komponente (C) ein Organohydrogenpolysiloxan ist, dargestellt durch die folgende Formel (1), 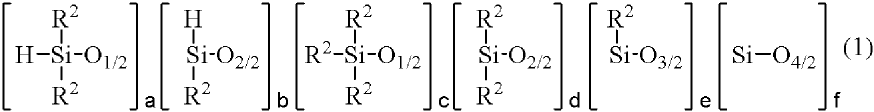 wobei jedes R₂ identisch oder voneinander verschieden ist und eine monovalente Kohlenwasserstoffgruppe mit 1 bis 10 Kohlenstoffatomen und keiner aliphatischen ungesättigten Bindung darstellt, "a" eine ganze Zahl von 0 oder mehr darstellt, "b" eine ganze Zahl von 1 oder mehr darstellt, "c" eine ganze Zahl von 0 oder mehr darstellt, "d" eine ganze Zahl von 0 oder mehr darstellt, "e" eine ganze Zahl von 0 oder mehr darstellt, und "f" eine ganze Zahl von 0 oder mehr darstellt, wobei a+b≥3 und 3≤a+b+c+d+e+f≤1000.

4. Die additionsreaktionshärtbare Silikonklebstoffzusammensetzung zur Hautanwendung gemäß einem der Ansprüche 1 bis 3, wobei die Komponente (A) in einer solchen Menge vorliegt, dass das Massenverhältnis von (A1)/(A2) in einem Bereich von 75/25 bis 25/75 liegt.

5. Die additionsreaktionshärtbare Silikonklebstoffzusammensetzung zur Hautanwendung gemäß einem der Ansprüche 1 bis 4, wobei die Komponente (X) ein Kondensationsreaktionsprodukt aus den Komponenten (A1), (A2), und (B), oder ein Gemisch aus der Komponente (A1) mit einem Kondensationsreaktionsprodukt aus den Komponenten (A2) und (B) ist.

6. Die additionsreaktionshärtbare Silikonklebstoffzusammensetzung zur Hautanwendung gemäß einem der Ansprüche 1 bis 5, ferner umfassend einen Additionsreaktionsinhibitor als Komponente (F) in einer Menge von 0,05 bis 8 Masseteilen relativ zu 100 Masseteilen der Summe der Komponenten (A) und (B).

7. Klebeband zur Hautanwendung, umfassend:
ein Substrat; und
eine gehärtete Produktschicht, die auf zumindest eine Seite des Substrats laminiert ist, wobei
die gehärtete Produktschicht aus einem gehärteten Produkt der Zusammensetzung gemäß einem der Ansprüche 1 bis 6 gebildet ist.

8. Verwendung des Klebebandes zur Hautanwendung nach Anspruch 7, umfassend das Anbringen des Klebebandes zur Hautanwendung auf der Haut.

## Revendications

1. Composition adhésive de silicone durcissable par réaction d'addition pour application cutanée, comprenant les constituants (X), (C) et (D) suivants :
(X) 100 parties en masse d'un mélange du constituant (A) suivant avec le constituant (B) suivant, ou d'un produit de réaction de condensation entre le constituant (A) suivant et le constituant (B) suivant,
(A) 55 à 90 parties en masse d'un diorganopolysiloxane constitué des constituants (A1) et (A2) suivants, dans lequel un rapport massique de (A1)/(A2) se situe dans une plage de 90/10 à 15/85,
(A1) un diorganopolysiloxane ayant deux groupes alcényle ou plus par molécule et une teneur en groupes alcényle d'au moins 0,0005 mol/100 g et inférieure à 0,15 mol/100 g, et dans lequel les extrémités de chaîne moléculaire ne sont pas complètement bloquées en ce sens que certains groupes SiOH subsistent, et
(A2) un diorganopolysiloxane ayant un groupe SiOH à une extrémité et aucun groupe alcényle, et
(B) 10 à 45 parties en masse d'un organopolysiloxane, à condition qu'un total des constituants (A) et (B) soit de 100 parties en masse, dans lequel l'organopolysiloxane contient un motif R¹₃SiO_{0,5}, un motif SiO₂ et un motif siloxane ayant un groupe hydroxyle lié à un atome de silicium et/ou un motif siloxane ayant un groupe alcoxy lié à un atome de silicium avec 1 à 6 atomes de carbone, chaque R¹ représente indépendamment un groupe hydrocarboné monovalent ayant 1 à 10 atomes de carbone, et un rapport molaire des motifs R¹₃SiO_{0,5}/SiO₂ est de 0,5 à 1,5 ;
(C) un organohydrogénopolysiloxane contenant trois groupes SiH ou plus par molécule, dans lequel l'organohydrogénopolysiloxane est présent en une quantité telle qu'un rapport molaire des groupes SiH dans le constituant (C) aux groupes alcényle dans le constituant (A) est de 0,2 à 15 ; et
(D) un catalyseur à base de métal du groupe du platine en une quantité de 1 à 5 000 ppm en termes de métal du groupe du platine par rapport à la quantité totale des constituants (A) et (B) sur la base de la masse.

2. Composition adhésive de silicone durcissable par réaction d'addition pour application cutanée selon la revendication 1, comprenant en outre le constituant (E) suivant en une quantité de 25 à 900 parties en masse par rapport à 100 parties en masse du total des constituants (A) et (B),
(E) au moins un solvant hydrocarboné aliphatique choisi dans le groupe constitué par l'hexane, l'heptane, l'isooctane, l'octane, le décane, le cyclohexane, le méthylcyclohexane et l'isoparaffine avec un point d'ébullition allant de 115 °C à 138 °C.

3. Composition adhésive de silicone durcissable par réaction d'addition pour application cutanée selon la revendication 1 ou 2, dans laquelle le constituant (C) est un organohydrogénopolysiloxane représenté par la formule (1) suivante, 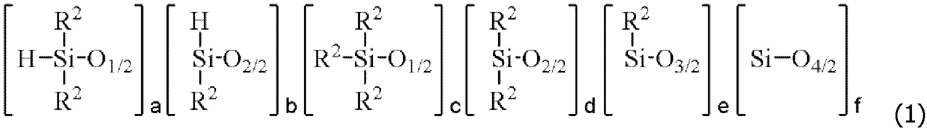 dans laquelle chaque R² est identique ou différent des autres et représente un groupe hydrocarboné monovalent ayant 1 à 10 atomes de carbone et aucune liaison aliphatique insaturée, « a » représente un nombre entier de 0 ou plus, « b » représente un nombre entier de 1 ou plus, « c » représente un nombre entier de 0 ou plus, « d » représente un nombre entier de 0 ou plus, « e » représente un nombre entier de 0 ou plus, et « f » représente un nombre entier de 0 ou plus, avec a+b ≥ 3 et 3 ≤ a+b+c+d+e+f ≤ 1 000.

4. Composition adhésive de silicone durcissable par réaction d'addition pour application cutanée selon l'une quelconque des revendications 1 à 3, dans laquelle le constituant (A) est présent en une quantité telle que le rapport massique (A1)/(A2) se situe dans une plage de 75/25 à 25/75.

5. Composition adhésive de silicone durcissable par réaction d'addition pour application cutanée selon l'une quelconque des revendications 1 à 4, dans laquelle le constituant (X) est un produit de réaction de condensation parmi les constituants (A1), (A2) et (B), ou un mélange du constituant (A1) avec un produit de réaction de condensation entre les constituants (A2) et (B).

6. Composition adhésive de silicone durcissable par réaction d'addition pour application cutanée selon l'une quelconque des revendications 1 à 5, comprenant en outre un inhibiteur de réaction d'addition en tant que constituant (F) en une quantité de 0,05 à 8 parties en masse par rapport à 100 parties en masse du total des constituants (A) et (B).

7. Ruban adhésif pour application cutanée, comprenant :
un substrat ; et
une couche de produit durci, stratifiée sur au moins un côté du substrat, dans laquelle la couche de produit durci est formée d'un produit durci de la composition selon l'une quelconque des revendications 1 à 6.

8. Utilisation du ruban adhésif pour application cutanée selon la revendication 7, comprenant la fixation du ruban adhésif pour application cutanée sur la peau.
